# EUROPÄISCHE PATENTSCHRIFT

(11) **EP 1 049 794 B1**
(45) Veröffentlichungstag und Bekanntmachung des Hinweises auf die Patenterteilung: **09.04.2003**
(21) Anmeldenummer: 98963424.1
(22) Anmeldetag: 09.11.1998
(51) Int. Cl.: C12P 13/00, C12P 1/00

(54) **VERFAHREN ZUR HERSTELLUNG VON L-CARNITIN AUS CROTONOBETAIN**
METHOD FOR PRODUCING L-CARNITINE FROM CROTONOBETAINE
PROCEDE DE PRODUCTION DE L-CARNITINE A PARTIR DE CROTONOBETAINE

(30) Priorität: 08.11.1997 DE 19749480
(43) Veröffentlichungstag der Anmeldung: 08.11.2000
(73) Patentinhaber: SIGMA-TAU Industrie Farmaceutiche Riunite S.p.A., 00144 Roma (IT)
(72) Erfinder: KLEBER, Hans-Peter, D-04416 Grossdeuben (DE); CANOVAS-DIAZ, Manuel, E-30001 Santo Angel-Murcia (ES); OBON, José Maria, E-30009 Murcia (ES); IBORRA, José Luis, E-30008 Murcia (ES)
(74) Vertreter: Spadaro, Marco
(86) Internationale Anmeldenummer: EP9807124
(87) Internationale Veröffentlichungsnummer: WO99024597

(56) Entgegenhaltungen:
- EP-A- 0 320 460
- CHEMICAL ABSTRACTS, vol. 115, no. 1, 8. Juli 1991 Columbus, Ohio, US; abstract no. 6956, JUNG, H. ET AL: "Production of L(-)- carnitine by biotransformation" XP002096268 & DECHEMA BIOTECHNOL. CONF. (1990), 4(PT. B, LECT. DECHEMA ANNU. MEET. BIOTECHNOL. 8TH, 1990), 1041-4 CODEN: DBCOEU,
- CHEMICAL ABSTRACTS, vol. 117, no. 19, 9. November 1992 Columbus, Ohio, US; abstract no. 187989, JUNG, HEINRICH ET AL: "L-(-)- Carnitine synthesis by stereoselective hydration of crotonobetaine" XP002096269 & PROC. - EUR. CONGR. BIOTECHNOL., 5TH (1990), VOLUME 1, 251-4. EDITOR(S): CHRISTIANSEN, CLAUS;MUNCK, LARS; VILLADSEN, JOHN. PUBLISHER: MUNKSGAARD, COPENHAGEN, DEN. CODEN: 57RVAO,
- CHEMICAL ABSTRACTS, vol. 128, no. 2, 12. Januar 1998 Columbus, Ohio, US; abstract no. 12603, OBON, JOSE MARIA ET AL: "L(-)- Carnitine production with immobilized Escherichi coli cells in continuous reactors" XP002096270 & ENZYME MICROB. TECHNOL. (1997), 21(7), 531-536 CODEN: EMTED2;ISSN: 0141-0229,

## Beschreibung

Die Erfindung betrifft ein Verfahren zur Herstellung von L-Carnitin aus. Crotonobetain, aus Salzen des Crotonobetains, anderen Derivaten des Crotonobetains oder dergleichen.

Es ist bekannt, daß L-Carnitin, eine ubiquitär vorkommende Verbindung, eine wichtige Rolle im Stoffwechsel - speziell beim Transport langkettiger Fettsäuren über die innere Mitochondrienmembran - spielt. Aus der Funktion des Carnitins im Stoffwechsel von Eukaryonten leiten sich zahlreiche klinische Anwendungen, z.B. in der Behandlung von Patienten mit Carnitinmangelsyndromen, in der Prophylaxe und Therapie verschiedener Herzkrankheiten sowie bei der Behandlung von Hämodialysepatienten, ab. Darüber hinaus ist L-Carnitin als Ergänzungsnährstoff von Bedeutung und fördert als Zusatz zu Fermentationsmedien auch das Wachstum von Hefen und Bakterien. Der wachsende Bedarf an diesem biologisch aktiven L-Carnitin-Enantiomer für diese und andere Anwendungen hat zu einer weltweiten Suche nach Synthesewegen für dieses Betain in einer optisch reinen Form geführt, da daß chemisch synthetisierte Racemat auf Grund der Hemmung der Carnitinacetyltransferase als auch des Carnitincarrierproteins nicht verwendet werden kann.

Zur Isolierung des L-Isomeren werden bis heute Verfahren genutzt, die auf der Racematspaltung mittels fraktionierter Kristallisation unter Anwendung optisch aktiver Säuren beruhen (z.B. US Patent 4, 254,053.1981), wobei D(+)-Carnitin als Abfallprodukt anfällt. Dieses Problem kann durch verschiedene biologische Verfahren, ausgehend von billigen achiralen Vorstufen (Adv. Biochem. Eng. Biotechnol., 1993, 50, 21-44) überwunden werden. Von speziellem Interesse ist die stereospezifische Hydration von trans-Crotonobetain zu L-Carnitin unter Anwendung von Stämmen der Genera *Escherichia* (ED 0121444.1984; DD 221 905.1987; ; EP 0320460.1989) oder *Proteus* (Agric. Biol. Chem., 1988, 52, 2415-2421: US Patent 5. 300. 430. 1994). Der Vorteil dieser Methode besteht darin, daß diese achirale Vorstufe auch durch chemische Dehydration des Abproduktes D-Carnitin erhalten werden kann.

CHEMICAL ABSTRACTS, vol. 115, no. 1, 8. Juli 1991 Columbus, Ohio, US; abstract no. 6956, JUNG, H. ET AL: 'Production of L(-)- carnitine by biotransformation' XP002096268 & DECHEMA BIOTECHNOL. CONF. (1990), 4(PT. B, LECT. DECHEMA ANNU. MEET. BIOTECHNOL. 8TH, 1990), 1041-4 CODEN: DBCOE offenbart ein Verfahren zur Herstellung von L-Carnitin, ausgehend von Crotonobetain unter Verwendung von immobilisierten Escherichia Coli 00K74-Zellen

CHEMICAL ABSTRACTS, vol. 117, no. 19, 9. November 1992 Columbus, Ohio, US; abstract no. 187989, JUNG, HEINRICH ET AL: 'L-(-)- Carnitine synthesis by stereoselective hydration of crotonobetaine' XP002096269 & PROC. - EUR. CONGR. BIOTECHNOL., 5TH (1990), VOLUME 1, 251-4. EDITOR(S): CHRISTIANSEN, CLAUS;MUNCK, LARS; VILLADSEN, JOHN. PUBLISHER: MUNKSGAARD, COPENHAGEN, DEN. CODEN: 57RVAO offenbart ein Verfahren zur Herstellung von L-Carnitin, ausgehend von Crotonobetain unter Verwendung von immobilisierten Escherichia Coli-Zellen.

Die zahlreich in der Literatur beschriebenen Verfahren mit immobilisierten Mikroorganismen in einem kontinuierlich arbeitenden Reaktorsystem haben den Vorteil, daß
- reinere Reaktionsmedien verwendet werden können und so die Extraktion sowie der Reinigungsprozeß erleichtert wird,
- durch Einsatz höherer Konzentrationen des Biokatalysators im Reaktionsmedium höhere Produktivitäten erreicht und zugleich die Möglichkeit von Kontaminationen vermindert werden,
- es zu einer Verminderung der Empfindlichkeit gegenüber Inhibitoren oder eines Nährstoffmangels kommt,
- eine höhere Stabilität des Biokatalysators erreicht wird.

Die genannten Vorteile können auch in einem kommerziell genutzten Prozeß verwendet werden.

Ein kontinuierlich arbeitender Reaktor, in dem Mikroorganismen durch Mikro- bzw. Ultrafiltrationsmembranen zurückgehalten werden, ist ein Immobilisierungsverfahren, welches neben den vorstehend genannten Vorteil auch den niedriger Kosten für die Immobilisierung besitzt und zugleich eine sehr leichte Maßstabsvergrößerung ermöglicht.

Entsprechend zielt die Erfindung auf ein Verfahren zur Produktion von L-Carnitin aus Crotonobetain, Crotonobetainsalzen oder anderen Crotonobetainderivaten in einem kontinuierlichen Reaktor mit freien oder immobilisierten Zellen, wachsenden oder ruhenden Zellen von *Escherichia coli* 044K74 (DSM 8828), die durch Mikro- bzw. Ultrafiltrationsmembranen in Ceramics, glasperlen oder Polymethansdreiben zurückgehalten werden.

*E. coli* wird in dem genannten Reaktor bei Temperaturen zwischen 20 und 40 °C, pH-Werten zwischen pH 6,0 - 8,0 und unter anaeroben Bedingungen, die für die Induktion der Carnitin-metabolisierenden Enzyme erforderlich sind, gehalten. Als Reaktionsmedien wird ein Minimal- oder Komplexmedium verwendet. In beiden Fällen wird Crotonobetain, Crotonobetainsalze oder andere Crotonobetainderivate in Konzentrationen zwischen 25 mmol und 1 M hinzugesetzt. Das Minimalmedium enthält unterschiedliche Konzentrationen von Caseinhydrolysat und Salzen (NH₄)₂SO₄, KH₂PO₄, K₂HPO₄, MgSO₄ x 7H₂O, MnSO₄ x 4H₂O, FeSO₄ x 7H₂O), während das Komplexmedium unterschiedliche Konzentrationen von pankreatischen Pepton und NaCI enthält. Zur Verbesserung des Wachstums von *E. coli* werden Glycerin, Glucose, Ribose, Saccharose oder Lactose hinzugesetzt. Zusätzlich werden dem Medium Inhibitoren, die die Transformation von Crotonobetain zu γ-Butyrobetain verhindern (Fumarat, Glucose oder Nitrat) und Induktoren Carnitin-metabolisierende Enzyme wie D-, L-, DL-Carnitin, ihre Salze und Derivate oder Crotonobetain, dessen Salze bzw. Derivate hinzusetzt.

Der Reaktionsablauf in dem hier verwendeten kontinuierlichen Zell-Recycle-Reaktor kann in zwei Abschnitte unterteilt werden. Der eine Abschnitt besteht aus einem Reaktortank, in welche Zellen von *E. coli* zusammen mit dem Reaktionsmedium den größten Teii des Crotonobetains in L-Carnitin umwandeln. Dieser Reaktortank besitzt Kontrollelemente für pH-Wert, Temperatur und Rührgeschwindigkeit sowie für die Kontrolle und Korrektur der Sauerstoffkonzentration. Die Zufuhr des Reaktionsmediums in den Reaktor erfolgt mittels einer Dosierpumpe. Wenn notwendig, muß eine Entlastung des Reaktortanks von überschüssigen Medium durchgeführt werden. Der zweite Abschnitt besteht aus einer externen Rückführungsschleife, die an den Reaktortank angeschlossen ist und über eine Pumpe den Inhalt des Reaktors durch eine Filtereinheit leitet. Während das Filtrat gesammelt wird, um L-Carnitin als Reaktionsprodukt daraus zu isolieren, wird der Rückstand aus der Filtration dem Reaktor wieder zugeführt. Zur Filtration der Zellsuspension können kommerzielle Filtersysteme unterschiedlicher Herkunft verwendet werden, solange sie eine Porengröße besitzen, die unterhalb der Zellgröße von *E. coli* liegt. Die Geschwindigkeit der Rückführpumpe bleibt unverändert, um best mögliche Filtrationsraten zu erreichen und um die Bildung einer Polarisationsmembran während des Filtrationsprozesses zu minimieren.

Der Ausdruck freie *E. coli* Zellen bezeichnet den Zustand, bei welchem ganze Zellen im Reaktionsmedium suspendiert sind, ohne daß ein Zellausfluß durch die Austrittslösung verhindert wird. Der Ausdruck immobilisierte Zellen beschreibt den Zustand, bei dem ganze Zeilen an lösliche Polymere oder unlösliche Träger gebunden sind, bzw. in Membransystemen einschlossen sind (In Methods in Enzymol. 1987. Vol. 135, 3-30).

Der Begriff Wachstumsbedingungen ist definiert als die Situation, in der ganze Zellen während ihres Lebenszyklus Substrate verbrauchen und Produkte bilden. Unter ruhenden Zellen versteht man intakte aber nicht wachsende Zellen, die unter bestimmten Bedingungen spezielle Stoffwechselleistungen zeigen (In "Biotechnology" (Kieslich, K.; Eds. Rehm, H.J. und Reed, G.) Verlag Chemie, Weinheim, Germany. 1984. Vol. 6a, 5-30).

Das Verfahren wird im Folgenden an einigen Ausführungsbeispielen erläutert:

### Beispiel 1:

*Escherichia coli* 044 K74 wird in einem bis zum Rand gefüllten, luftdicht verschlossenen Erlenmeyerkolben bei 37 °C unter anaeroben Bedingungen auf einem Rotationsschüttler (150 r.p.m.) kultiviert. Das verwendete Komplexmedium hat folgende Zusammensetzung: 50 mM Crotonobetain, 50 mM Fumarat, 5 g/l NaCI und verschiedene Konzentrationen (zwischen 0,5 und 10 g/l) pankreatisches Pepton. Der pH wird mittels KOH auf pH 7,5 eingestellt. In Tabelle 1 sind die spezifischen Wachstumsraten bei unterschiedlichen Peptonkonzentrationen zusammengefaßt.

**Tab. 1.**

| Maximale spezifische Wachstumsrate von *Escherichia coli* 044 K74 | | | | | |
|---|---|---|---|---|---|
| Peptone (g/l) | 0.5 | 1.0 | 2.5 | 5.0 | 10.0 |
| µmax (h⁻¹) | 0.224 | 0.296 | 0.351 | 0.372 | 0.325 |

Unter den genannten Bedingungen sind wachsende Zellen von *E coli* in der Lage, bis zur Beendigung des Versuches 20 - 30 mM L-Carnitin zu produzieren.

Konzentrationen höher als 5g/l Pepton ergeben sowohl ähnliche Wachstums- und kinetische Parameter als auch Biomassegehalte (OD 600 nm). Im Gegensatz dazu werden bei niedrigeren Peptonkonzentrationen geringere Wachstumsparameter erhalten.

### Beispiel 2:

*Escherichia coli* 044 K74 wird in einem bis zum Rand gefüllten, luftdicht verschlossenen Erlenmeyerkolben bei 37 °C unter anaeroben Bedingungen auf einem Rotationschüttler (150 r.p.m.) kultiviert. Das verwendete Komplexmedium hat folgende Zusammensetzung: 50 mM Crotonobetain, 5 g/l pankreatisches Pepton, 5 g/l NaCI sowie abgestufte Konzentrationen (zwischen 0 und 75 mM) Fumarat. Der pH wird mit KOH auf pH 7,5 eingestellt.

Tabelle 2 zeigt, daß ein Zusatz von Fumarat höhere Wachstumsraten von *E. coli* 044 K74 und eine OD bei 600 nm von nahezu 1,0 im steady state bedingt. Darüber hinaus bewirkt Fumarat eine L-Carnitinbildung von 20 - 30 mM bis zum Versuchsende. In Abwesenheit von Fumarat wird eine Carnitinkonzentration von nur 5 mM erhalten.

**Tabelle 2.**

| Biomasse (OD _{600 nm}) bei unterschiedlicher Fumaratkonzentration nach 10 h Versuchsdauer. | | | | |
|---|---|---|---|---|
| Fumarat(mM) | 0 | 25 | 50 | 75 |
| µmax (h⁻¹) | 0.21 | 0.37 | 0.38 | 0.39 |
| Biomasse OD (600 nm) | 0.980 | 0.910 | 1.00 | 0.950 |

### Beispiel 3:

Die Fähigkeit von *Escherichia coli* 044 K74, L-Carnitin aus Crotonobetain zu bilden, wird durch Crotonobetain induziert. Die Induktionsstudien wurden mit Crotonobetain zwischen 5 und 75 mM unter Verwendung von Ruhezelien durchgeführt. Bei hohen Crotonobetainkonzentrationen werden Umwandlungsraten von über 60 % L-Carnitin erreicht (Tabelle 3).

**Tabelle 3.**

| Abhängigkeit der L-Carnitinproduktion durch ruhende Zellen von *Escherichia coli* 044 K74 in Abhängigkeit von verschiedenen Crotonobetainkonzentrationen | | | | |
|---|---|---|---|---|
| Crotonobetain (mM) | 5 | 25 | 50 | 75 |
| L-Carnitin produktion (%) | 55 | 60 | 62 | 65 |

### Beispiel 4:

*Escherichia coli 044* K74 wird in einem bis zum Rand gefüllten, luftdicht verschlossenen Erlenmeyerkolben bei 37 °C unter anaeroben Bedingungen auf einem Rotationschüttler (150 r.p.m.) kultiviert. Das verwendete Komplexmedium hat folgende Zusammensetzung: 50 mM Crotonobetain, 5 g/l pankreatisches Pepton, 5 g/l NaCl und 50 mM Fumarat. Der pH wird mit KOH auf 7,5 eingestellt.

Um die Biokatalysatorkonzentation im Reaktor zu erhöhen und um eine. L-Carnitinproduktion bei Verdünnungsraten höher als die maximale spezifische Wachstumsrate zu ermöglichen, wurde ein Membranreaktor verwendet. Die Zellen wurden durch eine Polysulfon-Mikrofiltrationsmemban mit einer Ausschlußgrenze von 0,1 µm zurückkgehalten und wiederholt verwendet. Die Membranen waren in einem Plattenmodul angeordnet. In Tabelle 4 ist der Biomassegehalt und in Tabelle 5 die Carnitinproduktion, die Crotonobetainumwandlung und die Produktivität in diesem System zusammengefaßt.

**Tabelle 4.**

| Biomassegehalt von *E. coli* 044 K74 in einem kontinuierlich arbeitenden Membran-Reaktors | | | | |
|---|---|---|---|---|
| Verdünnungsrate (h⁻¹) | 0.0 | 0.2 | 1.0 | 2.0 |
| Biomasse (g_{Trockengewicht}/l) | 0.5 | 2.1 | 9.4 | 27.0 |

**Tabelle 5.**

| L-Carnitinproduktion, Crotonobetainumwandlung und Produktivität in einem .kontinuierlich arbeitenden des Zell-Reaktor mit *Escherichia coli* 044 K74 | | | | | |
|---|---|---|---|---|---|
| Verdünnungsrate (h⁻¹) | 0.0 | 0.5 | 1.0 | 1.5 | 2.0 |
| L-Carnitin-Produktion (%) | 0.0 | 38 | 42 | 42 | 38 |
| Crotonobetainumwandlung (%) | 0.0 | 24 | 26 | 26 | 30 |
| Produktivität (g/lᵣₑₐₖₜₒᵣ/h) | 0.0 | 1.75 | 3.5 | 5.5 | 6.5 |

Aus den Tabellen ist ersichtlich, daß mit immobilisierten Zellen von *Escherichia coli* 044 K74 in einem Zell-Recycle-Reaktor eine L-Carnitinbildung aus Crotonobetain von 6,5/l/h mit einer Metabolisierungsrate von nahezu 40 % erreicht wird.

## Patentansprüche

1. Verfahran zur Herstellung von L-Carnitin aus Crotonobetain, **dadurch gekennzeichnet, daß** ein immobilisierter Mikroorganismus in einem kontinuierlich arbeitenden Zellreaktor den Ausgangsstoff Crotonobetain als solchen oder in Form seiner Salze oder Derivate zu L-Carnitin umsetzt, wobei als Trägermaterial fur den Mikroorganismus Ceramics, Glasperlen, Polyurethanscheiben verwendet werden.

2. Verfahren nach Anspruch 1, **dadurch gekennzeichnet, daß** der Mlkroorganismus E, coli 044 K74 (DSM 8828) ist.

3. Verfahren nach Anspruch 1 und 2, **dadurch gekennzeichnet, daß** E. coli an Trägermaterial immobilisiert wird, das seine Lebensfähigkeit nicht beeinträchtigt.

4. Verfahren nach Anspruch 1 bis 3, **dadurch gekennzeichnet, daß** das Reaktionsgemisch von Ceramics, welche den Mikroorganismus tragen, filtriert wird, in an sich bekannter Weise das L-Camitin abgetrennt und das Crotonobetain in den Kreislauf zurückgeführt wird.

5. Verfahren nach Anspruch 1 bis 4, **dadurch gekennzeichnet, daß** die Crotonobetainkonzentration im Reaktionsrnedium zwischen 25 mM und 1 M beträgt.

6. Verfahren nach Anspruch 1 bis 5, **dadurch gekennzeichnet, daß** dem Reaktor zufließenden üblichen kommerziellen Komplexmedium oder gut definierten E coli -Minimalmedium Elektronenakzeptoren der Atmung der Inhi-bitoren wie Fumarat, Nitrat, Sauerstoff, N-Oxide oder Glucose zugesetzt werden, die die Hydrierung des Crotonobetains zu γ-Butyrobetain verhindern.

7. Verfahren nach Anspruch 1 bis 6, dadurch gekennzeichnel, daß dem Minimalmedium Induktoren Carnitin-metabolisierender Enzyme wie L-, D-, oder DL-Carnitin, -derivaten und -salzen sowie Crotonobetain, -derivaten und -salzen zugesetzt werden.

8. Verfahren nach Anspruch 1, **dadurch gekennzeichnet, daß** *E.* coli 044 K74 auf einem Komplexmedium anaerob oder partiell anaerob kul-tiviert wird, wobei eine Fumaratkonzentration von 50 mM eingehalten wird.

9. Verfahren nach Anspruch 1, **dadurch gekennzeichnet, daß** E. Coli 044 K74 auf einem Komplexmedium anaerob oder partiell anaerob kulti-viert wird. wobei das Komplexmedium 50 mM Crotonobetain, 5 g/l pankreati-sches Pepton, 5 g/l NaCl und 50 mM Fumarat enthält, einen pH von 7,5 auf-weist, und die Reaktion in einem kontinuierlich arbeitenden Membranreaktor abläuft.

10. Verfahren nach Anspruch 1 bis 7, **dadurch gekennzeichnet, daß** die Rückführung der Bakterien in den Reaktor kontinuierlich unter Verwendung kommersielier Querstromfiltrations- oder Hohlfaser-Module, die aus an sich bekannten Ultra- oder Mikrofiltrations-Membranen unterschiedlicher chemischer Zusammensetzung wie Cellulose, Polysuilphon oder polysalphoniertem Polysulphon mit einer Ausschußgrenze von 300 klDa bzw. 0,211 bestehen, erfolgt.

11. Verfahren nach Anspruch 1 bis 9, **dadurch gekennzeichnet, daß** die Synthese unter anaeroben oder partiell anaeroben Bedingungen durchgeführt wird.

12. Verfahren nach Anspruch 1, **dadurch gekennzeichnet, daß** die Camitinproduktion in einem kontinuierlich arbeitenden Reaktor bei unterschiedlichen Verdünnungsraten, die durch zwei Pumpen, der Dosierund Filtrationspumpe, eingestellt und bei verschiedenen Rührgeschwindigkeiten und Biomasse- Konzentrationen durchgeführt wird.

13. Verfahren nach Anspruch 12, dadurch gekennzeichet, daß die Ausflußrate des Filtrationsstromes prozeßleitend kontrolliert wird.

## Revendications

1. Procédé de fabrication de L-carnitine à partir de crotonobétaïne, **caractérisé en ce qu'**un microorganisme immobilisé transforme, dans un réacteur cellulaire fonctionnant en continu, la matière de sortie crotonobétaïne, en tant que telle ou sous la forme de ses sels ou de ses dérivés, en L-carnitine, dans lequel on utilise comme matériel de support pour le microorganisme des céramiques, des perles de verre ou des disques de polyuréthane.

2. Procédé selon la revendication 1, **caractérisé en ce que** le microorganisme est E. coli 044 K74 (DSM 8828).

3. Procédé selon la revendication 1 et 2, **caractérisé en ce que** E. coli est immobilisé sur un matériel de support, qui n'altère pas sa viabilité.

4. Procédé selon la revendication 1 à 3, **caractérisé en ce que** le mélange réactionnel est filtré des céramiques qui portent le microorganisme, **en ce que** la L-carnitine est séparée d'une façon connue en soi et **en ce que** la crotonobétaïne est reconduite dans le cycle.

5. Procédé selon la revendication 1 à 4, **caractérisé en ce que** la concentration de crotonobétaïne dans le milieu réactionnel est comprise entre 25 mM et 1 M.

6. Procédé selon la revendication 1 à 5, **caractérisé en ce qu'**on ajoute des accepteurs d'électrons de la respiration des inhibiteurs, tels que le fumarate, le nitrate, l'oxygène, le N-oxyde ou le glucose, qui empêchent l'hydrogénation de la crotonobétaïne en -butyrobétaïne, au milieu complexe commercial usuel ou au milieu minimal d'E. coli bien défini, s'écoulant vers le réacteur.

7. Procédé selon la revendication 1 à 6, **caractérisé en ce qu'**on ajoute au milieu minimal des inducteurs d'enzymes métabolisant la carnitine comme la L-, la D-ou la DL-carnitine, leurs dérivés ou leurs sels, comme la crotonobétaïne, ses dérivés ou ses sels.

8. Procédé selon la revendication 1, **caractérisé en ce que** E. coli 044 K74 est cultivé sur un milieu complexe en anaérobie ou en anaérobie partielle, qui contient une concentration de fumarate de 50 mM.

9. Procédé selon la revendication 1, **caractérisé en ce que** E. coli 044 K74 est cultivé sur un milieu complexe en anaérobie ou en anaérobie partielle, dans lequel le milieu complexe contient 50 mM de crotonobétaïne, 5 g/l de peptone pancréatique, 5 g/l de NaCl et 50 mM de fumarate, présente un pH de 7,5, et **caractérisé en ce que** la réaction se déroule dans un réacteur membranaire qui travaille en continu.

10. Procédé selon la revendication 1 à 7, **caractérisé en ce que** le retour des bactéries dans le réacteur se produit de façon continue par l'utilisation de modules commerciaux de filtration de courant transversal ou de modules commerciaux de fibres creuses, qui sont constitués de membranes d'ultra- ou microfiltration, connues en soi, d'une composition chimique différente, comme le cellulose, la polysulfone ou la polysulfone polysulfonée avec une limite d'exclusion de 300 kDa, respectivement 0,2 µ.

11. Procédé selon la revendication 1 à 9, **caractérisé en ce que** la synthèse est conduite dans des conditions d'anaérobie ou d'anaérobie partielle.

12. Procédé selon la revendication 1, **caractérisé en ce que** la production de carnitine est effectuée dans un réacteur travaillant en continu à différents taux de dilution, qui sont obtenus via deux pompes, la pompe de dosage et de filtration, et à différentes vitesses de mouvement et concentrations de biomasse.

13. Procédé selon la revendication 12, **caractérisé en ce que** le taux d'écoulement du courant de filtration est contrôlé au cours du procédé.

## Claims

1. Process for producing L-camitine from crotonobetaine, **characterised in that** an immobilised microorganism converts the starting material crotonobetaine as such or in the form of its salts or derivatives to L-carnitine in a continuously operating cell reactor, wherein ceramics, glass beads, polyurethane discs are used as substrate for the microorganism.

2. Process according to claim 1, **characterised in that** the microorganism is E. coli 044 K74 (DSM 8828).

3. Process according to claim 1 and 2, **characterised in that** E. coli is immobilised on substrate which does not impair its viability.

4. Process according to claim 1 to 3, **characterised in that** the reaction mixture is filtered by ceramics which support the microorganism, the L-carnitine is separated off in a manner known per se and the crotonobetaine is returned to the circuit.

5. Process according to claim 1 to 4, **characterised in that** the crotonobetaine concentration in the reaction medium is between 25 mM and 1 M.

6. Process according to claim 1 to 5, **characterised in that** electron acceptors for respiration of the inhibitors, such as fumarate, nitrate, oxygen, N-oxides or glucose, which prevent hydrogenation of the crotonobetaine to form γ-butyrobetaine, are added to conventional commercial complex medium flowing to the reactor or well defined E. coli minimal medium.

7. Process according to claim 1 to 6, **characterised in that** inductors of carnitine-metabolising enzymes, such as L-carnitine, D-carnitine, or DL-carnitine, derivatives and salts as well as crotonobetaine, derivatives and salts, are added to the minimal medium.

8. Process according to claim 1, **characterised in that** E. coli 044 K74 is cultivated on a complex medium anaerobically or partially anaerobically, wherein a fumarate concentration of 50 mM is maintained.

9. Process according to claim 1, **characterised in that** E. coli 044 K74 is cultivated on a complex medium anaerobically or partially anaerobically, wherein the complex medium contains 50 mM of crotonobetaine, 5 g/ of pancreatic peptone, 5 g/l of NaCl and 50 mM of fumarate, has a pH of 7.5, and the reaction proceeds in a continuously operating membrane reactor.

10. Process according to claim 1 to 7, **characterised in that** the return of bacteria to the reactor takes place continuously using commercial cross-flow filtration modules or hollow-fibre modules, which comprise ultrafiltration or microfiltration membranes of different chemical composition known per se, such as cellulose, polysulphone or polysalphonated polysulphone having an exclusion limit of 300 kDa or 0.211.

11. Process according to claim 1 to 9, **characterised in that** the synthesis is carried out under anaerobic or partially anaerobic conditions.

12. Process according to claim 1, **characterised in that** the carnitine production is carried out in a continuously operating reactor at different dilution rates, which are set by two pumps, the metering pump and filtration pump, and at different stirring rates and biomass concentrations.

13. Process according to claim 12, **characterised in that** the discharge rate of the filtration stream is controlled to manage the process.
